# EUROPEAN PATENT SPECIFICATION

(11) **EP 3 452 034 B1**
(45) Date of publication and mention of the grant of the patent: **15.05.2024**
(21) Application number: 17713314.7
(22) Date of filing: 30.03.2017
(51) Int. Cl.: A61K 31/27, A61P 9/10

(54) **CALPAIN INHIBITORS IN THE PREVENTION AND/OR TREATMENT OF VENTRICULAR REMODELLING**
CALPAIN-INHIBITOREN IN DER VORBEUGUNG UND/ODER BEHANDLUNG VON VENTRIKULÄRER NEUMODELLIERUNG
INHIBITEURS DE LA CALPAÏNE DANS LA PRÉVENTION ET/OU LE TRAITEMENT DE REMODELAGE VENTRICULAIRE

(30) Priority: 06.05.2016 EP 16168581
(43) Date of publication of application: 13.03.2019
(73) Proprietor: Fundació Hospital Universitari Vall d'Hebron - Institut de Recerca, 08035 Barcelona (ES); Senju Pharmaceutical Co., Ltd., Osaka 5410048 (JP)
(72) Inventor: GARCÍA DORADO GARCÍA, Antonio David, 08017 BARCELONA (ES); INSERTE IGUAL, Javier, 08107 MARTORELLES (ES); PONCELAS NOZAL, Marcos, 08028 BARCELONA (ES)
(74) Representative: ZBM Patents - Zea, Barlocci & Markvardsen
(86) International application number: PCT/EP2017/057501
(87) International publication number: WO 2017/190894

(56) References cited:
- WO-A1-2005/056519
- RU-C1- 2 526 466
- CHRISTIANE NEUHOF: "Calpain system and its involvement in myocardial ischemia and reperfusion injury", WORLD JOURNAL OF CARDIOLOGY, vol. 6, no. 7, 1 January 2014 (2014-01-01) , page 638, XP055310611, ISSN: 1949-8462, DOI: 10.4330/wjc.v6.i7.638
- TINGQIAO YE ET AL: "Over-Expression of Calpastatin Inhibits Calpain Activation and Attenuates Post-Infarction Myocardial Remodeling", PLOS ONE, vol. 10, no. 3, 18 March 2015 (2015-03-18) , page e0120178, XP055310616, DOI: 10.1371/journal.pone.0120178
- SEE F ET AL: "265 p38 mitogen-activated protein kinase inhibition prevents early adverse ventricular remodelling in the immediate post-myocardial infarction setting", EUROPEAN HEART JOURNAL, OXFORD UNIVERSITY PRESS, GB, vol. 24, no. 5, 1 March 2003 (2003-03-01), page 26, XP004528896, ISSN: 0195-668X, DOI: 10.1016/S0195-668X(03)93739-0
- ST JOHN SUTTON M G ET AL: "Left ventricular remodeling after myocardial infarction: pathophysiology and therapy", CIRCULATION, LIPPINCOTT WILLIAMS & WILKINS, US, vol. 101, 27 June 2000 (2000-06-27), pages 2981-2988, XP002714260, ISSN: 0009-7322, DOI: 10.1161/01.CIR.102.12.E9024
- Y. YOSHIKAWA ET AL: "A cardioprotective agent of a novel calpain inhibitor, SNJ-1945, exerts 1 actions on left ventricular mechanical work and energetics", AMERICAN JOURNAL OF PHYSIOLOGY: HEART AND CIRCULATORY PHYSIOLOGY, vol. 299, no. 2, 1 August 2010 (2010-08-01), pages H396-H401, XP055310565, US ISSN: 0363-6135, DOI: 10.1152/ajpheart.00153.2010
- Hiroshi Iwamoto ET AL: "Calpain Inhibitor-1 Reduces Infarct Size and DNA Fragmentation of Myocardium in Ischemic/Reperfused Rat Heart", Journal of Cardiovascular Pharmacology, 1 January 1999 (1999-01-01), pages 1-14, XP055690034, Retrieved from the Internet: URL:https://journals.lww.com/cardiovascula rpharm/Fulltext/1999/04000/Calpain_Inhibit or_1_Reduces_Infarct_Size_and_DNA.10.aspx [retrieved on 2020-04-28]
- Santhosh K Mani ET AL: "Calpain inhibition preserves myocardial structure and function following myocardial infarction", Am J Physiol Heart Circ Physiol., 1 January 2009 (2009-01-01), pages H1744-H1751, XP055690043, Retrieved from the Internet: URL:https://www.ncbi.nlm.nih.gov/pmc/artic les/PMC2781387/?report=printable [retrieved on 2020-04-28]
- MARCOS PONCELAS ET AL: "Delayed, oral pharmacological inhibition of calpains attenuates adverse post-infarction remodelling", CARDIOVASCULAR RESEARCH, vol. 113, no. 8, 1 July 2017 (2017-07-01), pages 950-961, XP055689336, GB ISSN: 0008-6363, DOI: 10.1093/cvr/cvx073
- BAINES ET AL.: "LOSS OF CYCLOPHILIN D REVEALS A CRITICAL ROLE FOR MITOCHONDRIAL PERMEABILITY TRANSITION IN CELL DEATH", NATURE, vol. 434, 1 March 2005 (2005-03-01), pages 658-662, DOI: doi:10.1038/nature03434
- NAKAGAWA ET AL: "CYCLOPHILIN D-DEPENDENT MITOCHONDRIAL PERMEABILITY TRANSITION REGULATES SOME NECROTIC BUT NOT APOPTOTIC CELL DEATH", NATURE, vol. 434, 31 March 2005 (2005-03-31), pages 652-658, DOI: doi:10.1038/nature03317
- CHEN-SCARABELLI ET AL.: "Cyclosporine A Prior to Primary PCI in STEMI Patients: The Coup de Grâce to Post-Conditioning?", JOURNAL OF THE AMERICAN COLLEGE OF CARDIOLOGY, 25 January 2016 (2016-01-25), pages 375-378, DOI: 10.1016/j.jacc.2015.11.024
- BERGMAN ET AL.: "Cardiac matrix metalloproteinase-2 expression independently induces marked ventricular remodeling and systolic dysfunction", AM J PHYSIOL HEART CIRC PHYSIOL, 8 December 2006 (2006-12-08), pages H1847-H1860, DOI: 10.1152/ajpheart.00434.2006
- HUDSON ET AL.: "Effects of selective matrix metalloproteinase inhibitor (PG-116800) to prevent ventricular remodeling after myocardial infarction: results of the PREMIER (Prevention of Myocardial Infarction Early Remodeling) trial", J AM COLL CARDIOL, 21 June 2006 (2006-06-21), pages 15-20, DOI: 10.1016/j.jacc.2006.02.055

## Description

This application claims the benefit of European Patent Application 16168581.3 filed May 6, 2016.

The present invention relates to the field of medical approaches for cardiopathies. In particular to the field of cardiopathies in which a sustained stress of the myocardium takes place.

### BACKGROUND ART

Ventricular remodelling results as a response to myocardial stress accompanying many cardiopathies, such as ischemia, arterial hypertension, valve diseases, myocarditis and dilated cardiomyopathy. This initially compensatory process evolves to adverse ventricular remodelling if stress persists. As a consequence there is cardiomyocyte hypertrophy, pro-inflammatory cell infiltration and excessive collagen deposition in interstitial matrix of myocardium, causing functional impairment and chronic heart failure.

Documents of See et al.,"p38 mitogen-activated protein kinase inhibition prevents early adverse ventricular remodelling in the immediate post-myocardial infarction setting", EUROPEAN HEART JOURNAL, 2003, vol 24, Issue suppl.1, September 2003, pp.: 26 and of St John Sutton et al., "Left ventricular remodeling after myocardial infarction: pathophysiology and therapy", CIRCULATION, 2000, vol 101, pp.: 2981-2988 disclose small organic molecules respectively RWJ-67657, an imidazole derivative and ACE inhibitors (captopril, a pyrrolidine derivative and furosemide, a benzene sulfonamide derivative for use in the treatment of adverse ventricular remodelling.

Nowadays, there is not any specific treatment for the adverse ventricular remodelling, but some clinical trials have shown that Angiotensin-converting enzyme (ACE) inhibitors and aldosterone inhibitors leads to improved myocardial performance in established remodelling with clinical heart failure symptoms.

There is literature focused on the use of calpain inhibitors for reducing calpain mediated injury in models of ischemia/reperfusion and cardioplegia.

As examples of such literature it is to be mentioned document of Yoshikawa et al., "Cardioprotective effects of a novel calpain inhibitor SNJ-1945 for reperfusion injury after cardioplegic arrest", Am J Physiol Heart Circ Physiol-2010, vol 298, pp.: 643-651. This document discloses the effects of administration of SNJ-1945 on cardiac dysfunction induced by cardioplegic arrest. SNJ-1945 was added to the cardioplegic solution and during 60 min of reperfusion. Among other data, the end-systolic pressure at midrange left ventricular volume (ESPmILW) are detailed and also the systolic pressure-volume area (PVA). Authors concluded that the inhibitor prevents left remodelling can be extracted with the experimental scheme and the measures performed in it, since adverse ventricular remodelling is a different pathology.

SNJ-1945 ( also named ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester) and other alpha-ketoamide derivatives of a particular formula, are disclosed in PCT application with publication number WO2005056519. They are proposed as useful in diseases related to calpain selected from ischemic disease, immunologic disease, multiple sclerosis, Alzheimer's disease, osteoporosis, diseases caused by brain tissue damage, cataract, glaucoma, retinal disease, retinochoroiditis, posterior eyeball complications or a disease involving neovascularization. In the particular case of SNJ-1945, the compound is used in a retinal ischemic disorder in a rat model, as a particular disease related to calpain, and proposed oral administration schedule is of 15 minutes before ischemia takes place and once after release of ischemia.

In relation with the effects of calpain and calpain inhibition in models of ischemia/reperfusion, it is to be mentioned the document of Inserte et al., "Contribution of calpains to myocardial ischaemia/reperfusion injury", Cardiovascular Research - 2012, vol 96, pp.: 23-31. Inserte et al, summarize what is known to date about the role of calpain in the damage caused by ischemia and reperfusion. The document mentions the use of some calpain inhibitors (A-705253 and MDL-28170) that showed a cardioprotective effect if administered at the start (onset) of reperfusion. The document shows that different inhibitors have been able to reduce acute reperfusion injury when administered during early reperfusion. All assays referred by Inserte et al., relate to assays with transgenic animals in which calpain activity is permanently reduced. It is widely known that this can mask results or real effects.

One reference mentioned by Inserte et al. is Hernando et al., "Calpain translocation and activation as a pharmacological targets during myocardial ischemia/reperfusion", J Mol Cell Cardiol - 2010, vol 49(2), pp.: 271-279. In Hernando et al., it is disclosed that using the calpain inhibitor MDL-28170, intravenously and during the first minutes of reperfusion, infarct size (area of risk and necrotic) was reduced. These data are, however, not conclusive for ventricular remodelling.

Another reference mentioned by Inserte et al. is Khalil et al., "Calpain inhibition reduces infarct size and improves global hemodynamics and left ventricular contractility in a porcine myocardial ischemia/reperfusion model", Eur J Pharmacol - 2005, vol 528, pp.: 124-131. In this case, it was shown that intravenously administration of the calpain inhibitor A-705253 before initiating ischemia, and during a reperfusion of 6 hours, not only reduced infarct size, but also improved hemodynamic and contractile function in relation to other studies.

As above stated, any of these models do not teach or provide conclusive data for adverse ventricular remodelling. Therefore, although many of the negative effects due to ischemic processes in myocardium or in other organs have been studied, as well as the effects in case of cardioplegia, there is still a need of a specific treatment for the adverse ventricular remodelling. This adverse ventricular remodelling takes place, commonly, in sustained ischemic myocardium episodes (such as in case of a myocardium infarction). Neither, there are preventive protocols to avoid this adverse ventricular remodelling caused in non-ischemic cardiac pathologies, in which the myocardium is sustained (for a long period or chronically) under stress conditions, such as in case of arterial hypertension.

### SUMMARY OF THE INVENTION

Inventors have surprisingly found that the calpain inhibitor ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester can be administered in a prolonged (also termed herewith sustained or chronic) way with real positive effects and without causing damage in subjects suffering from or at risk of suffering adverse ventricular remodelling (AVR) caused for example due to a myocardium infarct (MI). Inhibitors are also effective for preventing said AVR in case of suffering pathologies causing a sustained myocardial stress, such as arterial hypertension. Therefore, exogenous administration of the calpain inhibitors suppose a real advantage for subjects suffering from or at risk of suffering AVR, even with this administration is not for once, but for a long period of time.

Thus, a first aspect of the invention is a calpain a calpain inhibitor or a pharmaceutically acceptable salt thereof, for use in the prevention and/or treatment of adverse ventricular remodelling caused by myocardial infarction, wherein the calpain inhibitor is ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester, or a pharmaceutically acceptable salt thereof, and it is for a more than one day administration after reperfusion onset of the myocardium.

((1S)-1-((((1S)-1-benzyl-2,3-dioxo-3-(cyclopropylamino)-propyl)amino)carbonyl)-3-methylbutyl)carbamic acid 5-methoxy-3-oxapentyl ester, is also known and abbreviated herewith as SNJ-1945. Its IUPAC name is 2-(2-methoxyethoxy)ethyl N-[(2S)-1-[[(2S)-4-(cyclopropylamino)-3,4-dioxo-1-phenylbutan-2-yl]amino]-4-methyl-1-oxopentan-2-yl]carbamate.

As above exposed, adverse ventricular remodelling is the pathologic condition with myocardium dysfunctionalities and in which it is observed cardiomyocyte hypertrophy, pro-inflammatory cell infiltration and excessive collagen deposition in interstitial matrix of myocytes due to stress prolongation initially present as a compensatory process.

This aspect may also be formulated as the use of ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester or a pharmaceutically acceptable salt or solvate thereof as defined above for the manufacture of a medicament for the treatment or prevention of AVR caused by myocardial infarction, and wherein the compound is for a more than one day administration after reperfusion onset of the myocardium..

Thus, exogenous calpain inhibitor ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester is for use in the prevention and/or treatment of AVR caused by myocardial infarction, and wherein the compound is for a more than one day administration after reperfusion onset of the myocardium.

Another aspect of the invention is a pharmaceutical composition comprising a therapeutically effective amount of ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester or a pharmaceutically acceptable salt or solvate thereof, together with pharmaceutically acceptable excipients or carriers, said excipients comprising carboxymethylcellulose in a solvent, particularly, wherein the percentage by weight of carboxymethylcellulose in relation to said solvent is from 0.3 % (w/w) to 1.5 % (w/w).

### BRIEF DESCRIPTION OF THE DRAWINGS

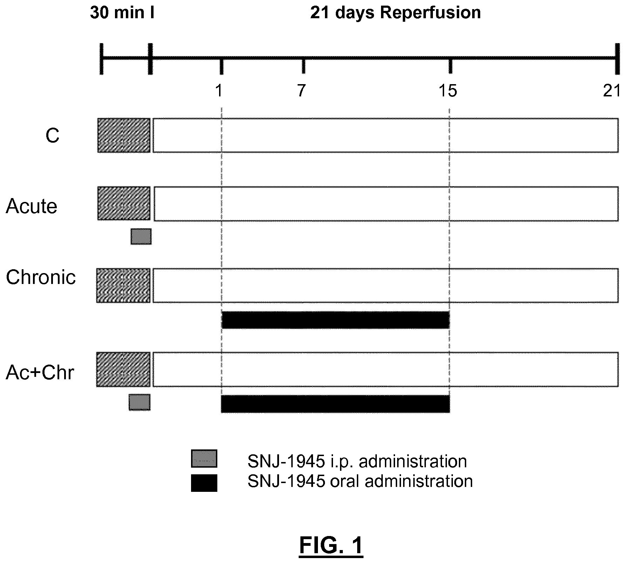

FIG. 1 shows the experimental protocol that was followed in an assay to test the ability of a calpain inhibitor (SNJ-1945) in the treatment of ventricular remodelling after a post myocardial infarction (post-MI) model (in Sprague-Dawley rats from Charles River) caused by transient ischemia. C means control (non-treated); Acute (if abbreviated, as Ac) means that SNJ-1945 was administered intraperitoneally during last minutes of coronary occlusion; Chronic (if abbreviated, as Chr) means that SNJ-1945 was orally administered (bolus with 120 mg/Kg SNJ-1945 in 0.5% carboxymethylcellulose) once daily, starting one day after reperfusion and for 14 days; Acute/Chronic (if abbreviated, as Ac+Chr) means that both types of administration were performed.

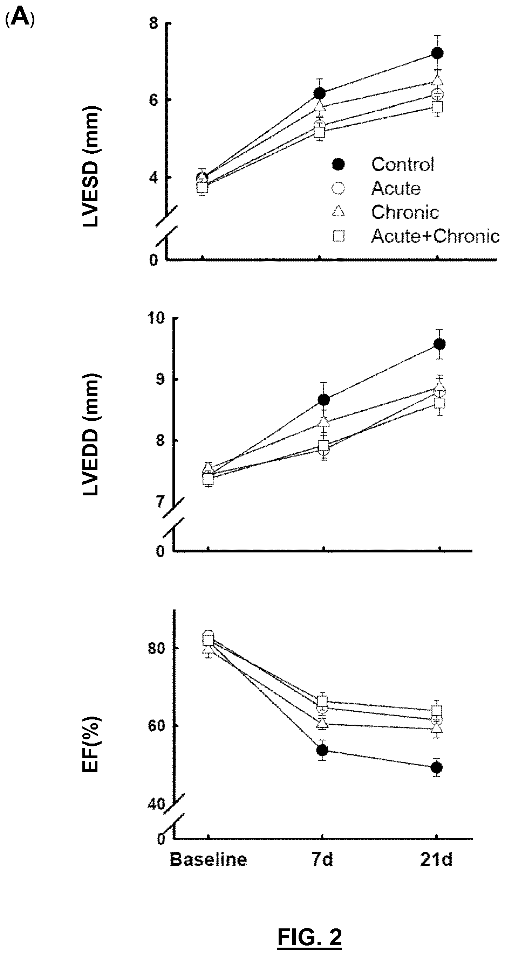

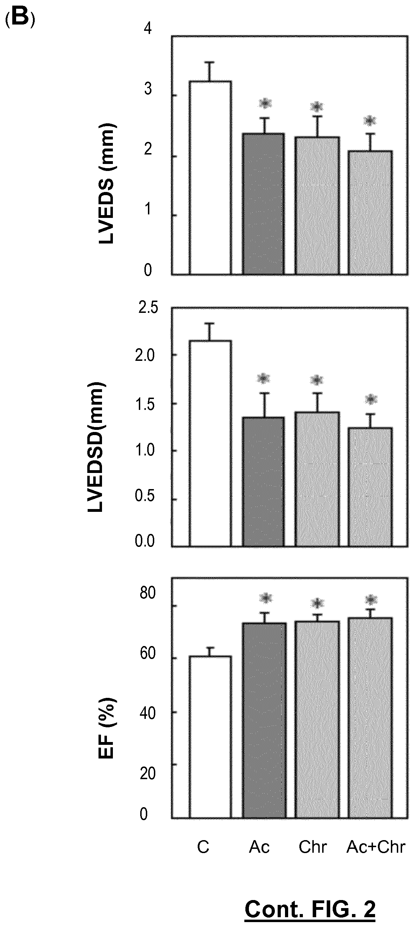

FIG. 2 shows different parameters analysed from an echocardiography in the different assayed groups disclosed in FIG. 1. In FIG. 2 (A), Control appears in black solid circles; Acute in white empty circles; Chronic in triangles and Acute+Chronic in squares. Data for each tested group at 21 days after reperfusion are depicted in FIG. 2 (B). LVEDD (in mm) is the Left ventricular end-diastolic diameter; LVESD (in mm) is Left ventricular end-systolic diameter; LVEDD and LVESD are the main echocardiographic parameters used for the evaluation of ventricular remodelling; EF (in percentage; %) is the Ejection fraction and determines the ventricular function. Measures where obtained at Baseline before MI, 7 days after reperfusion (7d) and 21 days after reperfusion (21d). (*p<0.05).

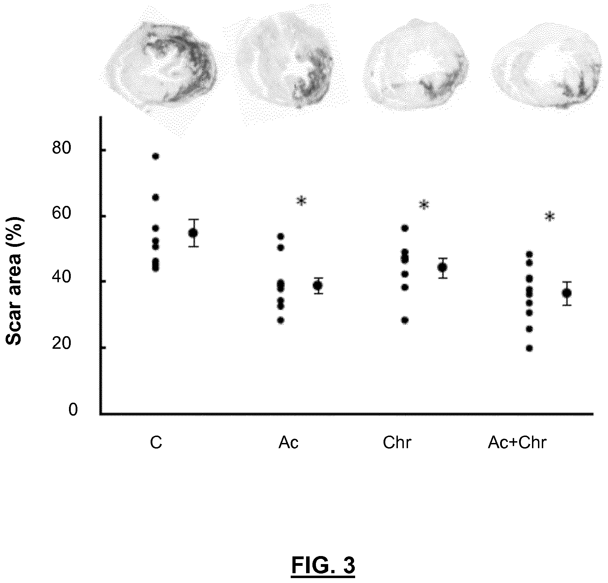

FIG. 3. Representative images from transversely sectioned heart slices showing the scar area stained with Pricosirius Red and its quantification expressed as a percentage of LV area in the different experimental groups detailed in FIG.1. Results are mean±S.E.M, n=9. *P<0.05 vs. control group. Abbreviations as indicated in FIG. 1.

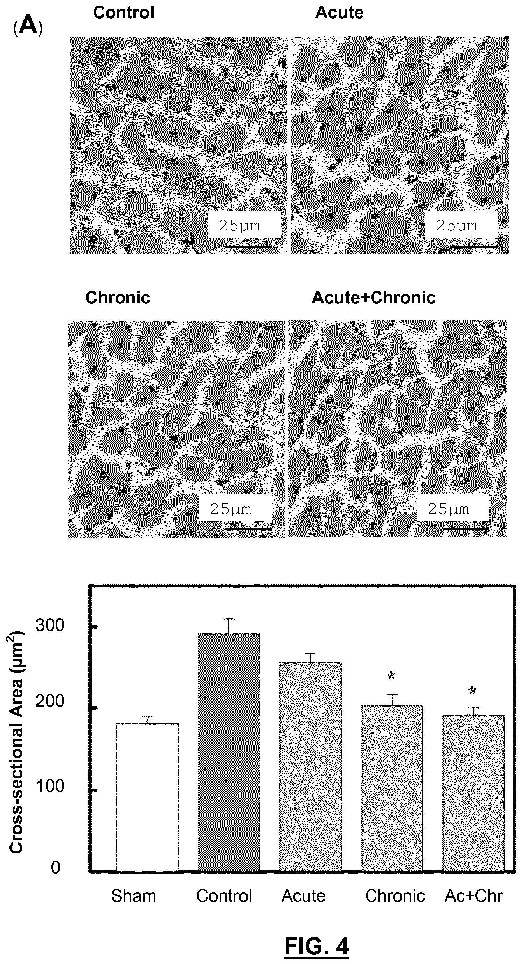

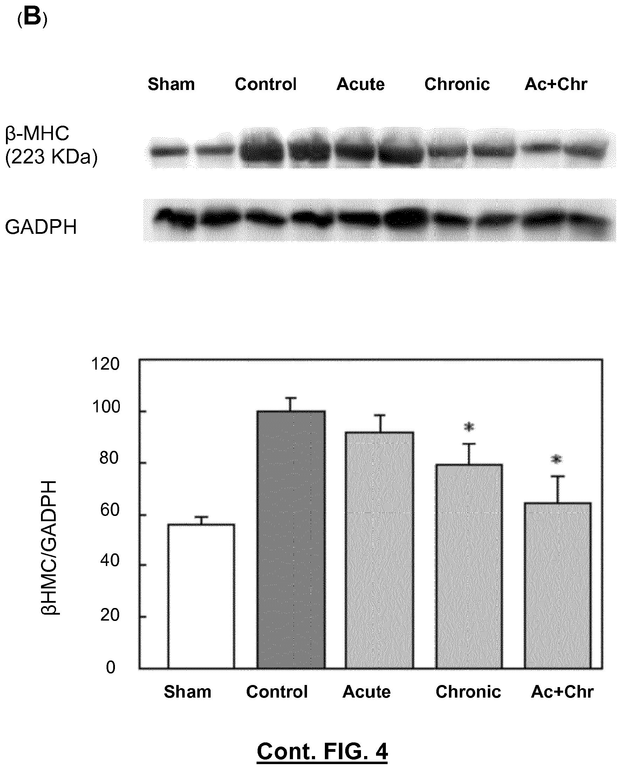

FIG. 4 depicts the cardiomyocyte hypertrophy effects in the non-ischemic myocardium. A) Representative ventricular cross-sections stained with hematoxylin & eosin corresponding to the non-infarcted area and quantification of cardiomyocyte cross-sectional area. At least 50 random cells from each heart (n=6 per group) were measured at 400x (Olympus Digital Camera C-1400L) with the NIH Image J 1.63 software. B) Representative western-blot of β-myosin heavy chain (β-HMC) (ABCAM) used as a marker of hypertrophy, and quantification of β-HMC protein relative to glyceraldehyde-3-phosphate dehydrogenase (GADPH) (ABCAM). Results are mean±S.E.M. *P<0.05 vs. control group. Sham relates to reference values of non-infarcted (non-operated) group. Abbreviations as indicated in FIG. 1.

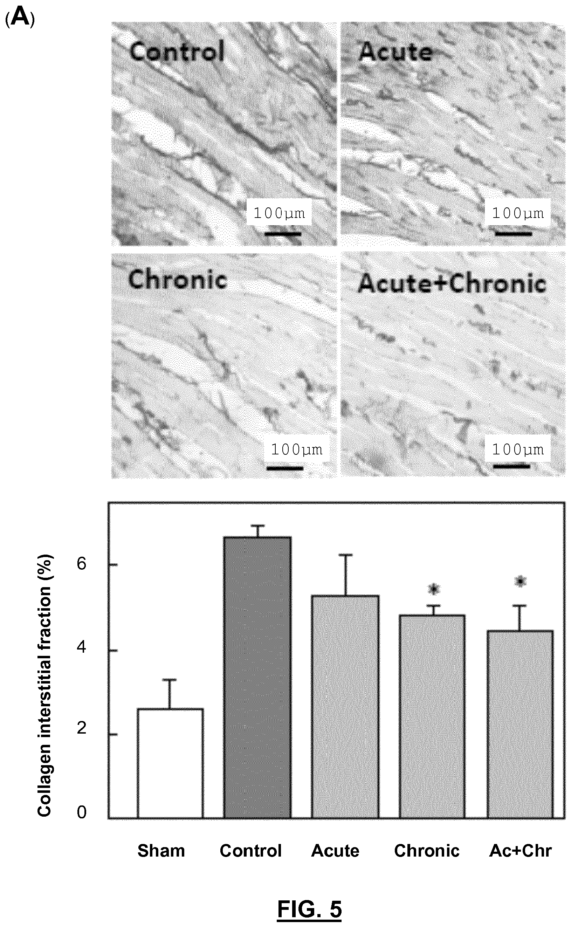

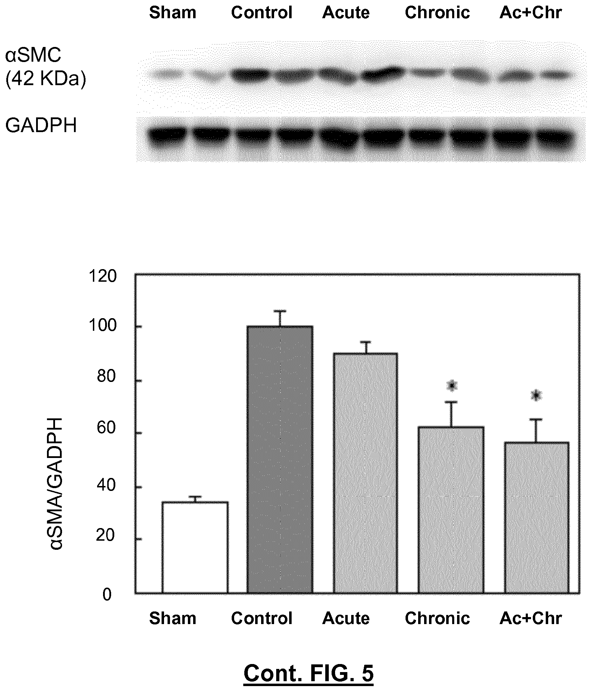

In FIG. 5 (A) Representative ventricular cross-sections stained with pricosirius red corresponding to the non-infarcted area obtained at 200x magnification (Olympus Digital Camera C-1400L) and bar diagram showing the quantification of interstitial collagen deposition of at least six random photomicrographs form each heart (n=6 per group). FIG. 5 (B) is a representative western-blot of α-smooth muscle actin (α-SMA, Sigma), used as a marker of fibroblast activation, and includes in a graphic of bars the quantification of α-SMA protein relative to GADPH (Abcam). Results are mean±S.E.M. *P<0.05 vs. control group. Sham relates to reference values of non-infarcted (non-operated) group. Abbreviations as indicated in FIG. 1.

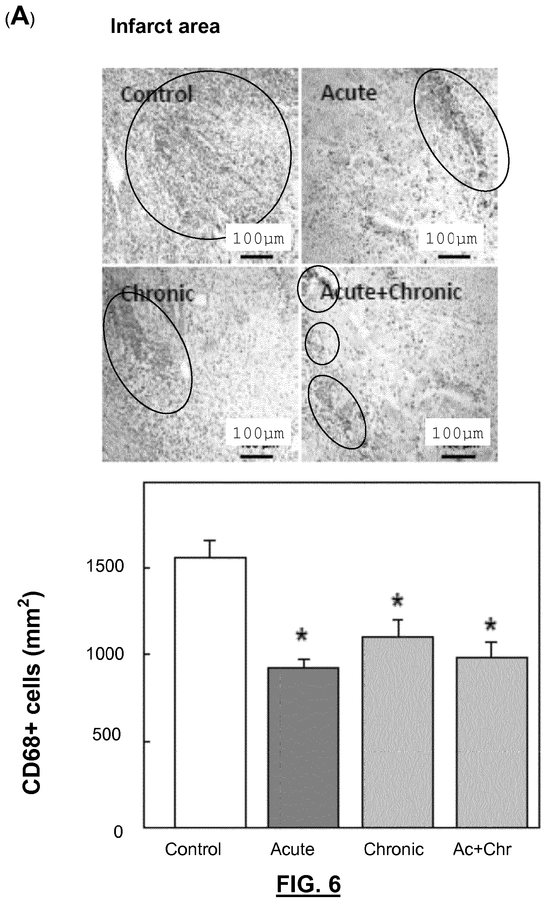

FIG. 6 (A) Representative ventricular cross-sections obtained after 3 days of reperfusion and immunostained against CD68 for monocytes/macrophages (AbD Serotec) and quantification of CD68+ cells in the infarcted area and (B) in the non-infarcted area (remote area). Results are mean±S.E.M. *P<0.05 vs. control group. Abbreviations as indicated in FIG. 1.

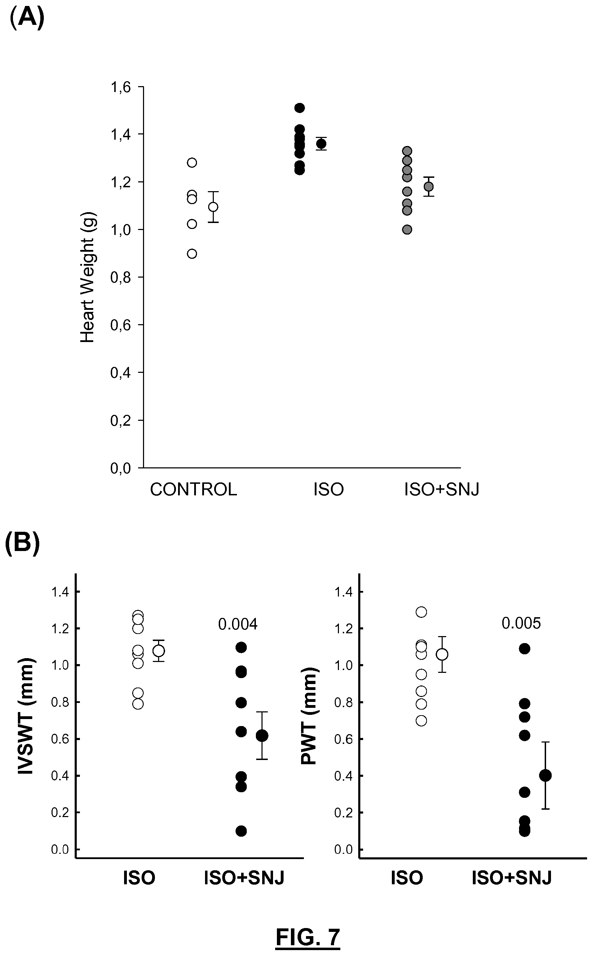

FIG. 7 (A), relating to Example 2 (comparative), is a graph with the heart weight (in grams; g) of Sprague-Dawley rats that received 5 mg/Kg/day isoproterenol (ISO) intraperitoneally for 1 week, of a control group not receiving isoproterenol (CONTROL), and of a group with co-administration of SNJ-1945 (orally) and isoproterenol (subcutaneous) for 7 consecutive days (ISO+SNJ). FIG. 7(B) shows the values of the interventricular wall (IVSWT) and posterior wall (PWT), measured by echocardiography, in ISO and ISO+SNJ groups.

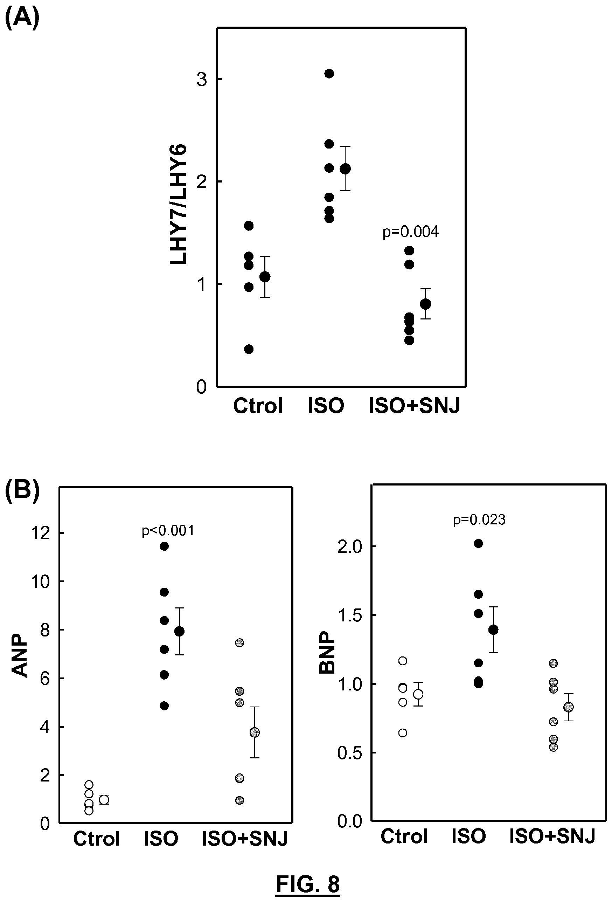

FIG. 8, relating to Example 2 (comparative), is a graph showing expression levels (amounts) of markers of hypertrophy measured by PCR in the assayed groups of rats as in FIG. 7. In particular, the ratio of beta- Myosin Heavy Chain (βMHC) and alpha Myosin Heavy Chain (αMHC), also termed as LHY7/LHY6 (FIG. 8(A)), and the expression of natriuretic peptides A (ANP) and B (BNP),(FIG. 8(B)). Ctrol. is the control.

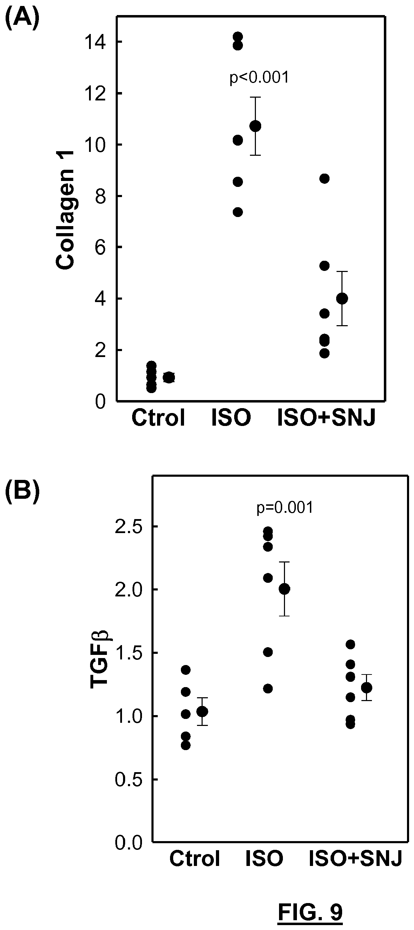

FIG. 9, also relating to Example 2 (comparative) and also for the three assayed groups as in FIGs. 7 and 8, shows collagen 1 levels (FIG. 9(A)) and the levels of tumoral growth factor β (TGFβ) (FIG. 9(B)), as markers of fibrosis, and all measured by PCR. Ctrol. is the control.

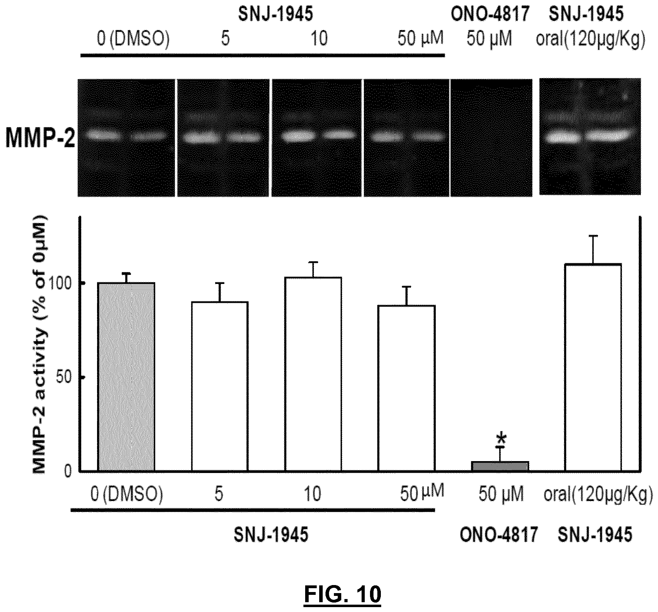

FIG. 10 is an image of gelatin zymograms of myocardial extracts obtained from control rats after 21 days of reperfusion incubated at 37°C overnight with DMSO (dimethylsulfoxide as vehicle), SNJ-1945 at the concentrations of 5, 10 and 50 µM and with the selective MMP inhibitor ONO-4817 ((*N*-[(1*S*,3*S*)-1-[(Ethoxymethoxy)methyl]-4-(hydroxyamino)-3-methyl-4-oxobutyl]-4-phenoxybenzamide)). Also data from oral gavage (120µg/Kg) of SNJ-1945 are depicted. MMP-2 means matrix metalloproteinase-2.

### DETAILED DESCRIPTION OF THE INVENTION

For "sustained myocardial stress" it is to be understood that myocardium is in a condition impairing its normal function due to a particular cause, in particular a pathology not necessarily stemming in the myocardium itself, but also due to abnormalities in coronary arteria and other vascular diseases. In any case, it is a damaging stress that derives from the persisting of some initially compensatory conditions for continuing ejecting blood. An example is the stress leading to adverse ventricular remodelling, wherein initially some parameters (at molecular and cell level) of the myocytes are altered for compensating a loss of function, such as the loss of other myocytes due to ischemia, said parameters evolving to a different pathological condition; the adverse ventricular remodelling.

The expression "sustained or prolonged use of a calpain inhibitor" means that said inhibitor is chronically administered, thus not only once. In the present invention is used as a synonymous of chronic administration. Thus for a particular period of time over one day, particularly 3 to 30 days, more particularly, from 7 to 21 days, and even more particularly from 7 to 14 days. Thus, it is used and usable for a more than one day administration, in particular according to a schedule prescribed by a facultative, which can be a daily schedule, and in particular for the rest of the subject life.

From "exogenous calpain inhibitor" or "calpain inhibitor" (used as synonymous) is to be understood as any substance able to reduce calpain activity and that is given to a subject, as an external element, in any pharmaceutical form. When the inhibitor is of peptide nature, it is externally administered in the peptide or protein form or even in form of an expression vector coding for said peptide or protein. "Exogenous calpain inhibitor" does not include the constitutive expression of a protein or peptide due to germline transgenic modifications in a genetic non-human experimental animal model.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "pharmaceutically acceptable salts" used herein encompasses any salt formed from pharmaceutically acceptable non-toxic acids including inorganic or organic acids. There is no limitation regarding the salts, except that if used for therapeutic purposes, they must be pharmaceutically acceptable.

The preparation of pharmaceutically acceptable salts of ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester can be carried out by methods known in the art. For instance, they can be prepared from the parent compound, which contains a basic or acidic moiety, by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them.

The compound of the invention may be in crystalline form either as free solvation compounds or as solvates (e.g. hydrates) and it is intended that both forms are within the scope of the present invention. Methods of solvation are generally known within the art.

According to the first aspect of the invention, adverse ventricular remodelling is caused by sustained myocardial stress in a pathology (namely a cardiopathy) caused by myocardial infarction.

Thus, the calpain inhibitor SNJ-1945 is for use in the treatment of adverse post-infarction ventricular remodelling (post-MI-AVR), and wherein the compound is for a more than one day administration after reperfusion onset of the myocardium. That is, CAD causing AVR is the sustained myocardial stress accompanying a myocardial infarct.

In another particular embodiment of the first aspect of the invention, the calpain inhibitor SNJ-1945 is for use chronically in the prevention and/or treatment of adverse ventricular remodelling. In particular, for a period from 3 to 30 days, more particularly, from 7 to 21 days, and even more particularly from 7 to 14 days. Chronically administered includes, according to the invention, continuously administered or intermittently for more than one day. Thus, it is used and usable for a more than one day administration, in particular the periods indicated above and more in particular a once daily administration.

In another particular embodiment, of the AVR caused by post-MI, a particular administration schedule comprises: administering the calpain inhibitor at appropriate dose the day of the myocardial infarct (before and/or after reperfusion has been started); and daily from 2 to 14 days. Most particularly, administration is started one day after reperfusion has taken place, and for a period from 2 to 14 days.

In the particular cardiopathies in which ischemia episodes are present, the calpain inhibitor is, in another particular embodiment, for use during said ischemic episode. This type of administration is named herewith as Acute administration; Acute or simply Ac. In a more particular embodiment, the calpain inhibitor is for use during said ischemic episode and after ischemic episode is finished and reperfusion has started. Administration after reperfusion is, in a particular embodiment, sustained performed, which means that it is continuously administered or intermittently for more than one day.

Another particular embodiment, of the first aspect of the invention, optionally in combination with any of the embodiments above or below, calpain inhibitor SNJ-1945 is for oral use in the prevention and/or treatment of AVR caused by myocardial infarction and as defined in the first aspect.

Oral use of the inhibitor is achieved in particular in the form of pharmaceutical compositions comprising an effective amount of calpain inhibitor SNJ-1945 or a pharmaceutically acceptable salt or solvate thereof, together with pharmaceutically acceptable excipients or carriers, said excipients comprising carboxymethylcellulose. Particular oral compositions comprise the inhibitor and carboxymethylcellulose in a solvent, particularly water, wherein the percentage by weight of carboxymethylcellulose in relation to said solvent is from 0.3 % (w/w) to 1.5 % (w/w). More in particular the percentage by weight of carboxymethylcellulose in relation to said solvent is from 0.5 % (w/w) to 1.0% (w/w). More in particular, 0.5 % (w/w). In another particular embodiment, optionally in combination with any of the embodiments above or below, the calpain inhibitor SNJ-1945 in the oral compositions is in an amount for a dosage from 50 mg/Kg to 300 mg/Kg, more in particular from 100 mg/Kg to 150 mg/Kg, and more in particular from 120 mg/Kg to 150 mg/Kg, all intervals including the extremes.

For being soluble in water, SNJ-1945 can be formulated either to be injected or to be orally administered for the use in the prevention and/or treatment of AVR caused by MI and as indicated in the first aspect.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1: SNJ-1945 for use in the prevention and/or treatment of adverse ventricular remodelling caused by myocardial infarction

In vivo myocardial infarction
Sprague-Dawley rats (250-300 g) were premedicated with atropine (0.05 mg/kg ip), anaesthetized with ketamine (75 mg/kg ip) and xylazine (10 mg/kg ip), and mechanically ventilated (Inspira ASV, Harvard Apparatus). Anaesthesia was maintained with 1-2% isoflurane and body temperature controlled with a heating pad to 36-37 °C. The heart was exposed through the fourth intercostal space and the left anterior descending coronary artery (LAD) ligated using 6/0 silk suture (Ethicon Endo-surgery, OH, USA), 1 mm distal to left atrial appendage. After occlusion for 30 min, the suture was loosened to start reperfusion and buprenorphine (0.05mg/kg per 6 h, subcutaneously) was given for 48 h. Mice with lack of ST-elevation during ischemia or lack of ST-recovery at reperfusion were excluded from further evaluation.

The effect of SNJ-1945 on post-infarct remodelling and heart function was analyzed in 48 mice reperfused for 21 days (n=12 per group). The different experimental groups are shown in FIG. 1. In the acute group, SNJ-1945 (120 mg/kg + 0.5% carboximethylcellulose) was administered intraperitoneally 5 min before starting reperfusion. In the chronic group, SNJ-1945 (120 mg/kg + 0.5% carboximethylcellulose) was administered orally over 14 days starting after 24h of reperfusion. The delayed administration of the drug allow us to determine whether calpain inhibition alters myocardial remodelling by a direct modulation of the mechanisms responsible of the process and not just by reducing the cell death occurring during the acute phase of reperfusion. In the last group, acute and chronic administration of SNJ-1945 were combined (Ac+Chr). The effective dose of SNJ-1945 was previously determined based on Trager N (Trager et al., "Effects of a novel orally administered calpain inhibitor SNJ-1945 on immunomodulation and neurodegeneration in a murine model of multiple sclerosis", J.Neurochem- 2014, vol. no. 130(2), pp.: 268-279) and unpublished studies on dose-dependent data from our laboratory.

### Echocardiographic analysis

Rats underwent transthoracic echocardiography at baseline (before surgery), and at 7 and 21 days after surgery. Echocardiography was performed using a Vivid portable ultrasound system with a i12L-RS 12MHz transducer (GE Healthcare) as described earlier (Poncelas, Inserte et al. 2015, "Obesity induced by high fat diet attenuates postinfarct myocardial remodeling and dysfunction in adult B6D2F1 mice", Journal of Molecular and Cellular Cradiology-2015, vol. no.84, pp.: 154-161). The left ventricular end-systolic (LVESd) and diastolic (LVEDs) internal diameters, interventricular septum thickness (IVS) and posterior wall thickness (LVPW) were measured in M-mode recordings. Left ventricular ejection fraction (LVEF) was calculated according to standard formulas.

### Histology and immunohistochemistry

After 21 days of reperfusion hearts were rapidly excised, weighed, fixed in buffered 4% paraformaldehyde and embedded in paraffin for histological evaluation. Sections at the papillary muscles level were cut at 4 µm and stained with Picrosirius red (Sigma-Aldrich, MO, USA) to visualize the area of scar and interstitial fibrosis. Digitalized images were evaluated using ImagePro Software (Image Pro-Plus, Media Cybernetics, MD, USA) and the area of scar was expressed as the percentage of the fibrotic area to left ventricular area as previously described (Poncelas, Inserte et al. 2015, *supra).* The analysis of interstitial collagen deposition was assessed in the posteroseptal non-infarcted myocardium where six random photomicrographs from each heart (n=6 per group) were taken at 200x magnification.

Mean cardiomyocyte cross-sectional area was measured in sections stained with hematoxylin and eosin. At least 50 random cells from each heart (n=6 per group) were measured at 400x magnification.

Macrophage infiltration in the non-infarcted myocardium was analyzed by immunohistochemistry in hearts reperfused for 3 days (n=4 per group). Myocardial sections were exposed to H2O2/methanol, levamisol (DakoCytomation) and avidin/biotin blocking (Vector Laboratories) to neutralize endogenous peroxidase, phosphatase and biotin, respectively. Sections were probed with CD68 (AbD Serotec) for monocytes/macrophages, incubated with a biotinylated secondary antibody (1 :200, ABC Peroxidase standard stainig kit, Thermo Scientific) in 2% normal blocking serum and visualized using 3, 3'-diaminobenzidine (DAB, Sigma). Six random photomicrographs from the infarct area and six from the non-infarcted area for each heart (n=6 per group) were taken at 200x magnification.

An investigator blinded to the treatment protocols performed the above analyses using NIH Image J 1.63 software.

### Western-blot

Heart samples were homogenized in buffer containing Tris-HCl 50 mmol/L, NaCl 150 mmol/L, EDTA 10 mmol/L, DTT 1 mmol/L, NaF 10 mmol/L, Na3VO4 2 mmol/L, 1% Triton X-100, and 1% Protease Inhibitor Cocktail (Sigma), pH 7.3, and centrifuged at 15 000 g for 15 min. Samples from supernatants were separated on 10% SDS-PAGE, transferred to nitrocellulose membranes (Hybond ECL, Amersham), blocked with 5% PhosphoBlocker Blocking Reagent (Cell Biolabs, Inc.). Primary antibodies used were raised against calpain-1 (Abcam), calpain-2 (Abcam), calpastatin (Abcam), α-fodrin (Enzo), β-MHC (Abcam), α-SMC (Sigma) and GAPDH (Genetex). Bands were detected by chemiluminescence (SuperSignal West Dura Extended Duration Substrate, Pierce) and quantified using a charge-coupled device system (Image Reader LAS-3000, Fujifilm) and an image analysis software (Image Gauge, Fujifilm).

### Statistical analysis

Data analysis was performed using SPSS for Windows. Means between groups were compared by one-way ANOVA or unpaired t-test. Least significant square test was applied as post hoc test when significant differences were observed. Repeated measures ANOVA and Dunnett's post hoc test were used to compare temporal differences in the echocardiographic. P<0.05 was considered to be statistically significant. In all cases, the assumption of normality was examined in SPSS before the statistical analysis using the Shapiro-Wilk test. When P>0.05 distribution of data was considered normal. All results are expressed as mean±S.E.M.

### RESULTS

Administration of SNJ-1945 in the acute or chronic phase attenuates cardiac remodelling and ventricular dysfunction

From the 48 mice subjected to ischemia and 21 days of reperfusion protocol 38 were included in the study. Mortality rate was 12.5% with no differences in survival rate between groups (4 mice during surgery and 2 mice at 12 and 24 h of reperfusion). Four animals were excluded for no clear confirmation of ST-during ischemia.

Baseline echocardiographic evaluation did not show any difference between groups. Myocardial infarction resulted in a progressive increase in LVEDD and LVESD and a decrease in the EF (FIG. 2). These changes were observed over time and occurred rapidly during the first week of reperfusion and continued more slowly thereafter. No significant changes in LVPW and IVS were reported. LV dilation and dysfunction were significantly attenuated in the groups receiving SNJ-1945 during the acute phase of reperfusion (Acute and Ac+Chr groups) when measured at 1 week of reperfusion compared to control mice and also in the chronic group at 21 days of reperfusion. No significant differences in echocardiographic parameters were observed between the acute and chronic treatment at the end of the experiment. In FIG. 2 (B) values of each parameter indicated with bars at 21 days for each group.

The scar size, defined as the area of LV stained with pricosirius red after 21 days of reperfusion was 34.6±4.2%. Both, intraperitoneal administration of SNJ-1945 at the time of reperfusion and oral administration starting after 24h of reperfusion attenuated the scar area, by 29.1% in the acute group (P=0.002), 22.7% in the chronic group (P=0.036) and 33.7% in the Ac+Chr group (P=0.003) (FIG. 3). Area of LV stained with pricosirius red is depicted in FIG. 3 as the black shadow in the transversely sectioned heart slices of each group. Besides mean of percentage area of each groups, as well as values of each individual appear in the graphic below the slices.

The combination of acute and chronic administration of SNJ-1945, although shows a trend towards a more effective attenuation of LV dilatation, increased ejection fraction and reduced scar area, did not result in a statistically significant improvement with respect the individual treatments.

### Chronic oral administration of SNJ-1945 limits cardiomyocyte hypertrophy

The cardiomyocyte cross-sectional area in the non-ischemic myocardium, indicative of hypertrophy, was increased in the control group respect to sham operated group. Chronic but not acute inhibition of calpains with SNJ-1945 significantly reduced cardiomyocyte hypertrophy (P=0.041 in the chronic group and P=0.021 in the Ac+Chr group) (FIG. 4 (A)). In FIG. 4(A) the ventricular cross-sections stained with hematoxylin & eosin corresponding to the non-infarcted area and the quantification of cardiomyocyte cross-sectional area (in µm²) in a graphic with bars. At least 50 random cells from each heart (n=6 per group) were measured at 400x (Olympus Digital Camera C-1400L) with the NIH Image J 1.63 software. Furthermore, β-MHC protein levels were increased in control group, providing further evidence of a hypertrophic phenotype, and attenuated only in groups treated chronically with SNJ-1945 (P=0.036 in the chronic group and P=0.019 in the Ac+Chr group) (FIG. 4(B)). In FIG. 4(B) there is represented the western-blot of β-HMC) (ABCAM) used as a marker of hypertrophy, and in a bar graphic the quantification of β-HMC protein relative to glyceraldehyde-3-phosphate dehydrogenase (GADPH) (ABCAM) No significant additive effect was observed when both treatments were combined.

Chronic oral administration of SNJ-1945 attenuates interstitial collagen deposition.

Myocardial infarction increased interstitial collagen deposition in the non-infarcted area in all groups. Chronic oral administration with SNJ-1945, but not acute treatment, reduced the amount of interstitial collagen (P=0.029 in the chronic group and P=0.109 in the acute group). No further reduction in interstitial fibrosis was observed when SNJ-1945 was given during the acute and chronic phase of reperfusion (P=0.016) (FIG. 5(A)). In line with these results, the levels of α-SMA, marker of fibroblast activation, were reduced in hearts receiving chronic treatment (P=0.012 in the chronic group and P=0.009 in the Ac+Chr group) (FIG. 5(B)).

Calpain inhibition attenuates inflammatory cell infiltration.

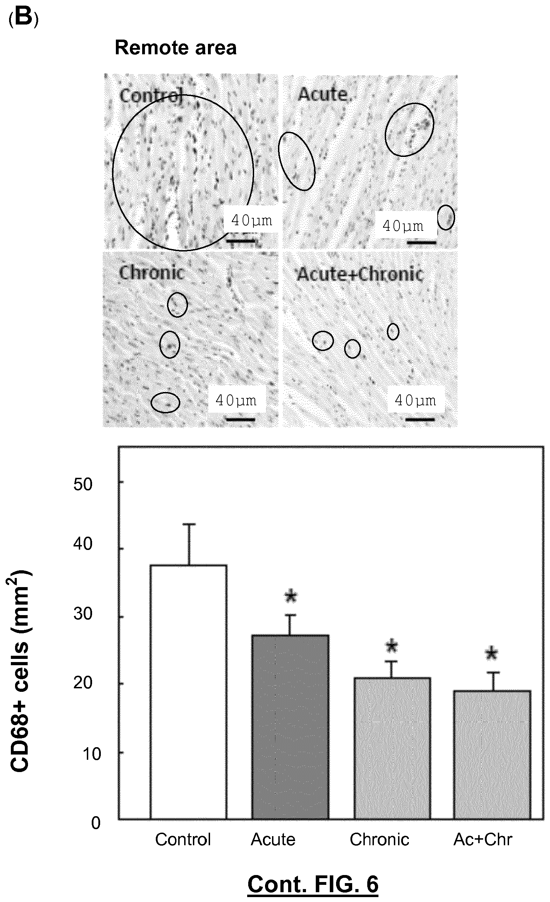

Inflammatory cell infiltration was determined in a subgroup of hearts reperfused for 3 days (n=4 per group). Previous results from our group and others (Christia et al., "Systematic Characterization of Myocardial Inflammation, Repair, and Remodeling in a Mouse Model of Reperfused Myocardial Infarction", Journal of Hystochemistry& Cytochemistry-2013, vol. no. 61(8), pp.: 555-570), indicate that at this day of reperfusion infiltration of macrophages corresponding to the proinflammatory M1 population is maximal and negligible after 21 days. The results shown in FIG. 6 demonstrate that both, acute and chronic calpain inhibition attenuate infiltration of monocytes/macrophages in the ischemic area (P=0.002 in the acute acute group and P=0.028 in the chronic group) and in the non-ischemic area (P=0.046 in the acute group and P=0.001 in the chronic group) with no significant additive effect when acute and chronic treatments were combined (P<0.001). In FIG. 6 (A) there are depicted ventricular cross-sections obtained after 3 days of reperfusion and immunostained against CD68 for monocytes/macrophages (AbD Serotec). Shadow areas (in a circle) correspond to staining with antibody (orange-green in colour images). Quantification of CD68+ cells in the infarcted area (as area of positive cells in mm²) is depicted for each group in the graphic with bars. In FIG. 6 (B), the same parameters as in (A) are depicted for the non-infarcted area (remote area). Results are mean±S.E.M. *P<0.05 vs. control group.

### CONCLUSIONS:

The present investigation provides evidence supporting pharmacological inhibition of calpains during reperfusion as a strategy to limit post-infarct myocardial remodelling and dysfunction. The results demonstrate that in addition to the cardioprotective effects of calpain inhibition at the time of reperfusion, the delayed and long-term oral administration of the calpain inhibitor SNJ-1945 attenuates ventricular remodelling and dysfunction by modulating the hypertrophy of cardiomyocytes, collagen deposition and infiltration of inflammatory cells in the non-infarcted myocardium.

### Example 2 (comparative). Effect of SNJ-1945 on remodelling of non-ischemic origin: effects on myocardial hypertrophy and fibrosis induced by isoproterenol

Chronic administration of isoproterenol is the most common small animal model in the study of myocardial remodeling. In orderto study the effect of calpain inhibition in AVR of a different cause than myocardial infarction, myocardial hypertrophy and fibrosis induced by isoproterenol.

The calpain inhibitor SNJ-1945 was co-administered orally once a day to male Sprague-Dawley rats that received 5 mg/Kg/day isoproterenol (ISO) intraperitoneally for 1 week. Assayed groups were as follows:
Isoproterenol group (ISO): administration of isoproterenol as a single daily subcutaneous injection for 7 consecutive days.
SNJ-1945 treated group (SNJ+ISO): co-administration of SNJ-1945 (orally) and isoproterenol (subcutaneous) for 7 consecutive days.

Hearts were obtained and compared with vehicle-treated and ISO treated rats not receiving the calpain inhibitor.

In FIG. 7, there are depicted individual values and mean values of the heart weight (FIG. 7(A)), the interventricular wall (IVSWT) and posterior wall (PWT) thickness (FIG. 7(B)) induced by isoproterenol and measured by echocardiography.

As depicted in this FIG. 7 (A and B), SNJ-1945 reduces the increase in heart weight (A) and in the interventricular wall (IVSWT) and posterior wall (PWT) thickness (B) induced by isoproterenol and measured by echocardiography. Echocardiography was performed using a Vivid portable ultrasound system with a i12L-RS 12MHz transducer (GE Healthcare) as described earlier (Poncelas, Inserte et al. 2015, "Obesity induced by high fat diet attenuates postinfarct myocardial remodeling and dysfunction in adult B6D2F1 mice", Journal of Molecular and Cellular Cardiology-2015, vol. no.84, pp.: 154-161).

In FIG. 8, there are depicted the expression levels (amounts) of markers of hypertrophy measured by PCR. In particular, the ratio LHY7/LHY6 (FIG. 8(A)), and the expression of natriuretic peptides A (ANP) and B (BNP), in FIG. 8(B), of the three assayed groups.

Further, in FIG. 9 and also for the three assayed groups, there are depicted the collagen 1 levels (FIG. 9(A)) and the levels of tumoral growth factor β (TGFβ) (FIG. 9(B)), as markers of fibrosis, all measured by PCR.

For the PCR, total RNA was extracted from ischemic and non-ischemic area of heart samples of the three assayed groups using TRIsureTM reagent (BIOLINE, UK) following the manufacturer's protocol. Quantitative RT-PCR was performed using Taqman universal PCR master mix (Applied Biosystems, USA). The amplification program consisted of 50°C for 2 minutes, 95°C for 10 minutes and 40 cycles of 95°C for 15 sec and 60°C for 1 minutes and was performed in a 7900HT Fast Real-Time PCR System (Applied Biosystems, USA). Primers used for ANP, BNP, collagen I, Myh6, Myh7, and TGF-B1 were purchased from Thermofisher and were those in the following Gene Expression Assays (Rn00561661_m1, Rn04219558_g1, Rn01463848_m1, Rn00691721_g1, Rn01488777_g1, and Rn00572010_m1, respectively, reference to assays retrievable from Thermofisher catalogue at March 1, 2017; https-//www.thermofisher.com/order/genome-database/details/gene-expression/).

At 7 days of ISO treatment, calpain-1 and calpain-2 were overexpressed and correlated with increased calpain activity (data not shown). Oral co-administration of SNJ-1945 attenuated calpain activation and as deducible from FIG. 7 (A and B) reduced heart weight, septal and left ventricular posterior wall thicknesses as determined by echocardiography, as well as cardiomyocyte crossectional area. Administration of SNJ-1945 reduced β-MHC/α-MHC ratio and ANP and BNP mRNA levels, as deducible from FIG. 8 (A and B). In addition, SNJ-1945 also significantly reduced the intraventricular collagen deposition and the expression of collagen I, and TGFβ induced by ISO, as derivable from FIG. 9 (A and B).

From all these data it can be concluded that chronic oral administration of SNJ-1945 attenuates isoproterenol-induced concentric cardiac hypertrophy and fibrosis. Pharmacological calpain inhibition is possible with oral therapy and represents an effective strategy to limit adverse ventricular remodeling of non-ischemic origin. The results demonstrate that chronic (more than one day) oral administration of SNJ-1945 prevents isoproterenol induced cardiac hypertrophy and fibrosis and extends the use of pharmacological calpain inhibition as an effective strategy to limit adverse ventricular remodeling of non-ischemic origin.

### Example 3. Dose-dependency and inhibitory specificity of SNJ1945

Studies performed in isolated rat hearts subjected to ischemia/reperfusion showed that perfusion with SNJ-1945 produces a dose-dependent reduction in calpain-specific α-fodrin breakdown products, reflecting calpain inhibition (data not shown). According to these data and the pharmacokinetic parameters of SNJ-1945, rats received orally (by gavage) the drug at the doses of 30, 60 or 120 mg/kg. In these experimental conditions, proteolisis of α-fodrin measured after 60 minutes of reperfusion was significantly reduced in rats treated with 120 mg/kg SNJ-1945. To discard the possibility that the effects of SNJ-1945 were consequence of its inhibitory action on matrix metalloproteinase-2 (MMP-2), the gelatinolytic activity of MMP-2 was measured by zymography in samples incubated with different concentrations of SNJ-1945.

Activity of MMP-2 was evaluated by gelatin zymography. Briefly, myocardial preparations obtained from rats reperfused for 21 days (control group) were run on a 10% polyacrylamide gel copolymerized with 1.5 mg/ml gelatin. After washing with 2.5% Triton X-100, gels were cut into strips and incubated separately, overnight, in zymography buffer with SNJ-1945 at the concentrations of 5, 10 and 50 µM, DMSO as a vehicle control or the selective MMP inhibitor ONO-4817 (*N*-[(1*S*,3*S*)-1-[(Ethoxymethoxy)methyl]-4-(hydroxyamino)-3-methyl-4-oxobutyl]-4-phenoxybenzamide) (50 µM, Tocris). It was further examined MMP-2 activity (as % of 0 µM) in samples prepared from animals receiving SNJ-1945 by oral gavage and reperfused for 21 days (Chronic group). Gels were stained in 0.25% Coomassie Brilliant blue G, 40% methanol, and gelatinolytic activities quantitated after destaining.

The results obtained are depicted in FIG. 10 and demonstrated that SNJ-1945 does not inhibit the MMP-2 activity at the concentrations tested, supporting that calpain inhibition is its primary mechanism of action._FIG. 10 shows the gelatin zymograms of the myocardial extracts obtained from control rats after 21 days of reperfusion incubated at 37°C overnight with DMSO (vehicle), SNJ-1945 at the concentrations of 5, 10 and 50 µM and with the selective MMP inhibitor ONO-4817. The zymogram at right corresponded to myocardial extracts from rats receiving SNJ-1945 administered orally over 14 days. Results are mean±S.E.M, n=3 per group.

SNJ-1945, is a third generation calpain inhibitor, specifically designed to increase its water solubility and bioavailability and less prone to react non-specifically with amino and thiol groups in contrast to other peptidyl inhibitors such leupeptin, calpeptin and SJA6017. Although it has been suggested that the substrates and actions of calpains and MMP-2 in various cellular pathways overlaps, and it has been demonstrated that some widely used calpain inhibitors also inhibit MMP-2, the results herewith rule-out the possibility that the effects obtained with SNJ-1945 were consequence of an inhibitory action on MMP-2.

### REFERENCES CITED IN THE APPLICATION

- See et al.,"p38 mitogen-activated protein kinase inhibition prevents early adverse ventricular remodelling in the immediate post-myocardial infarction setting", EUROPEAN HEART JOURNAL, 2003, vol 24, Issue suppl._1, September 2003, pp.: 26.- St John Sutton "Left ventricular remodeling after myocardial infarction: pathophysiology and therapy", CIRCULATION, 2000, vol 101, pp.: 2981-2988.
   - Yoshikawa et al., "Cardioprotective effects of a novel calpain inhibitor SNJ-1945 for reperfusion injury after cardioplegic arrest", Am J Physiol Heart Circ Physiol - 2010, vol 298, pp.: 643-651.
   - Inserte et al., "Contribution of calpains to myocardial ischaemia/reperfusion injury", Cardiovascular Research - 2012, vol 96, pp.: 23-31.
   - Hernando et al., "Calpain translocation and activation as a pharmacological targets during myocardial ischemia/reperfusion", J Mol Cell Cardiol - 2010, vol 49(2), pp.: 271-279.
- Khalil et al., "Calpain inhibition reduces infarct size and improves global hemodynamics and left ventricular contractility in a porcine myocardial ischemia/reperfusion model", Eur J Pharmacol - 2005, vol 528, pp.: 124-131.
- WO2005056519.
- Trager et al., "Effects of a novel orally administered calpain inhibitor SNJ-1945 on immunomodulation and neurodegeneration in a murine model of multiple sclerosis", J.Neurochem- 2014, vol. no. 130(2), pp.: 268-279.
- Poncelas, Inserte et al. 2015, "Obesity induced by high fat diet attenuates postinfarct myocardial remodeling and dysfunction in adult B6D2F1 mice", Journal of Molecular and Cellular Cradiology-2015, vol. no.84, pp.: 154-161.
- Christia et al., "Systematic Characterization of Myocardial Inflammation, Repair, and Remodeling in a Mouse Model of Reperfused Myocardial Infarction", Journal of Hystochemistry& Cytochemistry-2013, vol. no. 61(8), pp.: 555-570.

## Claims

1. A calpain inhibitor or a pharmaceutically acceptable salt thereof, for use in the prevention and/or treatment of adverse ventricular remodelling caused by myocardial infarction, wherein the calpain inhibitor is ((1S)-1-((((1S)-1-benzyl-2,3-dioxo-3-(cyclopropylamino)-propyl)amino)carbonyl)-3-methylbutyl)carbamic acid 5-methoxy-3-oxapentyl ester or a pharmaceutically acceptable salt thereof, and it is for a more than one day administration after reperfusion onset of the myocardium.

2. The calpain inhibitor for use according to claim 1, wherein the calpain inhibitor is for use during ischemic episode and after ischemic episode is finished and reperfusion of myocardium started.

3. The calpain inhibitor for use according to claim 1, wherein the administration of the calpain inhibitor is started one day after reperfusion has taken place, and for a period from 2 to 14 days.

4. The calpain inhibitor for use according to any of claims 1-3, wherein calpain inhibitor is for oral use.

## Patentansprüche

1. Ein Calpain-Inhibitor oder ein pharmazeutisch akzeptables Salz davon zur Verwendung bei der Prävention und/oder der Behandlung von unerwünschtem ventrikulärem Remodeling, das durch einen Myokardinfarkt verursacht worden ist, wobei der Calpain-Inhibitor das 5-Methoxy-3-oxapentylester von ((1S)-1-((((1S)-1-Benzyl-2,3-dioxo-3-(cyclopropylamino)-propyl)amino)carbonyl)-3-methylbutyl)carbaminsäure oder ein pharmazeutisch akzeptables Salz davon ist, und es ist für eine mehr als eintägige Verabreichung nach Reperfusionsbeginn des Myokards.

2. Der Calpain-Inhibitor zur Verwendung nach Anspruch 1, wobei der Calpain-Inhibitor zur Verwendung während einer ischämischen Episode und nachdem die ischämische Episode beendet ist und die Reperfusion des Myokards begonnen hat.

3. Der Calpain-Inhibitor zur Verwendung nach Anspruch 1, wobei die Verabreichung des Calpain-Inhibitors einen Tag begonnen wird, nachdem die Reperfusion stattgefunden hat und für einen Zeitraum von 2 bis 14 Tagen.

4. Der Calpain-Inhibitor zur Verwendung nach einem der Ansprüche 1 bis 3, wobei der Calpain-Inhibitor zur oralen Verwendung bestimmt ist.

## Revendications

1. Un inhibiteur de la calpaïne ou un sel pharmaceutiquement acceptable de celui-ci, destiné à être utilisé dans la prévention et/ou le traitement du remodelage ventriculaire indésirable causé par un infarctus du myocarde, dans lequel l'inhibiteur de la calpaïne est l'ester 5-méthoxy-3-oxapentylique de l'acide ((1S)-1-(((1 S)-1-benzyl-2,3-dioxo-3-(cyclopropylamino)-propyl)amino)carbonyl)-3-méthylbutyl)carbamique ou un sel pharmaceutiquement acceptable de celui-ci, et il est destiné à une administration de plus d'un jour après le début de la reperfusion du myocarde.

2. L'inhibiteur de la calpaïne pour l'utilisation selon la revendication 1, dans lequel l'inhibiteur de la calpaïne est destiné à être utilisé pendant un épisode ischémique et après la fin de l'épisode ischémique et le début de la reperfusion du myocarde.

3. L'inhibiteur de la calpaïne pour l'utilisation selon la revendication 1, dans lequel l'administration de l'inhibiteur de la calpaïne est commencée un jour après que la reperfusion a eu lieu et pendant une période de 2 à 14 jours.

4. L'inhibiteur de la calpaïne pour l'utilisation selon l'une quelconque des revendications 1 à 3, dans lequel l'inhibiteur de la calpaïne est pour l'utilisation orale.
